# EUROPEAN PATENT APPLICATION

(11) **EP 4 119 226 A1**
(43) Date of publication of application: **18.01.2023**
(21) Application number: 21185829.5
(22) Date of filing: 15.07.2021
(51) Int. Cl.: B01L 3/00, C12M 1/32, C12M 3/06, F16K 99/00

(54) **AN ASSAY PLATE SYSTEM TO ASSAY A CELL CULTURE MODEL**

(71) Applicant: Hooke Bio Ltd., V14XH92, Shannon, Co. Clare (IE)
(72) Inventor: Cliffe, Fionla, Co. Limerick, V94 DH6Y (IE); Lyons, Mark, Clare, V94 F8N3 (IE); Madden, Conor, Limerick, Raheen (IE); Keady, Tara, Galway, H91 XP1H (IE); Devitt, Shane, Co. Clare, V95 HP7R (IE); Costello, Patrick, Co. Galway, Oranmore (IE); Ramesh, Sumir, Co. Clare, Shannon (IE)
(74) Representative: Purdylucey Intellectual Property

(57) **Abstract**

An assay plate system to assay a cell culture model, comprises an assay plate (1) having an upper surface (5), a lower surface, at least first and second assay wells (37) each having an open top (39) in fluid communication with the upper surface and a transparent base (40) configured to allow imaging of the base of the well from under the plate body, and at least one fluidic conduit (12, 13) comprising a fluid inlet and a fluid outlet and configured to fluidically connect the fluid inlet, the first well, the second well and the fluid outlet in series. The upper surface of the assay plate comprises a recessed section (10, 11) having an elongated groove and a cover of resiliently deformable material (15, 30) covering the recess, whereby a first section of the fluidic conduit is defined between the elongated groove and a cover of resiliently deformable material. The first recess (10) of the assay plate comprises a plurality of first fluidic conduits (12) and a plurality of second fluidic conduits (13) arranged in an intersecting array, in which an assay well (37) is located at each point of intersection of the intersecting array of fluidic conduits.

## Description

### Field of the Invention

The present invention relates to an assay plate system to assay a cell culture model and especially a 3-D cell culture model such as an organoid or microtissue. Also contemplated is a method of assaying a cell culture model that employs an assay plate system of the invention.

### Background to the Invention

During the process of drug discovery, test drugs need to be tested on cells to determine how they work and the effects of the drug on the cells. To date, the pharmaceutical industry has relied primarily on animal models and human cell line cultures that bear little resemblance to normal or disease human tissue, resulting in only one in every ten drugs making it through clinical testing. This high failure rate in clinical trials of drugs that make it through pre-clinical testing adds greatly to the cost of drug development.

Microtissues or organoids are tiny, self-organized three-dimensional tissue cultures that are derived from stem cells. Such cultures can be crafted to replicate much of the complexity of an organ, or to express selected aspects of it like producing only certain types of cells. Organoids/microtissues may be grown from a variety of precursor cell types including stem cells-cells that can divide indefinitely and produce different types of cells as part of their progeny. Scientists have learned how to create the right environment for the precursor cells so they can follow their own genetic instructions to self-organize, forming tiny structures that resemble miniature organs composed of many cell types. Organoids/ microtissues can range in size from less than the width of a hair to 5 mm.

Many scientists believe that microtissues have the capacity to be more accurate and physiologically relevant models than existing animal models and that using organoids in pre-clinical testing of drugs will reduce the failure rate of drugs that enter clinical trials. These models will allow drug and vaccine manufacturers to bring products to market in a more timely and efficient manner. There is therefore a need for a technology to facilitate high throughput organoid screening that reproduces physiological conditions (e.g. temperature, CO2 and nutrients), facilitates fluid flow to the organoids, and allows organoids to be imaged.

It is an objective of the invention to overcome at least one of the above-referenced problems.

### Summary of the Invention

The applicant provides an assay plate system suitable for assaying in real-time cell culture models, especially 3-D cell culture models such as organoids, microtissues and spheroids, generally in a high-throughput manner. The system comprises a plate with assay wells and a fluidic system comprising fluidic conduits configured to provide assay fluid to the wells. To enable the use of plate with multiple wells and multiple fluidic conduits, the Applicant has a provided a plate with a recessed upper surface, wells provided in the recess each having an open top that fluidically communicates with the recess, and one or more elongated grooves formed in the surface of the recess. A cover of resiliently deformable material (e.g. silicone) is provided on top of the recess, thus providing one or more fluidic conduits in the recess defined by the elongated grooves and the cover layer of resiliently deformable material. The fluidic conduit provides fluid communication from a fluid inlet in the plate to the wells and to a fluidic outlet, in series. The fact that the wells are formed in the recess and the fluidic conduits are defined by elongated grooves also formed in the recess ensures that the fluidic conduits are fluidically sealed while allowing supply of assay fluid into the top of the wells, away from the cell culture model. In addition, the use of a recess, elongated grooves and a cover layer of resiliently deformable material to form the fluidic conduits, facilitates the fabrication of a plate with intersecting fluidic conduits and assay wells disposed at the point of intersection, enabling the provision of wells in a grid array format and the routing of assay fluid specifically along rows of wells or specifically along columns of wells - this allows assay fluid to be provided to a first well containing a first cell culture model, and then from the first well to a second well containing a second cell culture model, allowing dynamic cell-cell interplay to be monitored in real-time. In addition, the plate body system of the invention, and in particular the deformable fluidic conduits that are partly defined by a resiliently deformable material, allow the opening and closing of selected fluidic conduits using a fluid control module having actuation pins, which allows fluid to be routed across the plate in a desired manner, for example only along rows of wells (e.g. for assay fluid to be recirculated across wells having replicates of the same organoid type) or only along columns of wells (allowing assay fluid to be recirculated across well containing different cell culture model types). The assay plate may also have two recessed sections and two fluid control modules configured to act in sequence to pump assay fluid through a fluid recirculation conduit and continually recirculate assay fluid to the wells, thereby reproducing a physiological system.

In a first aspect, the invention provides an assay plate system to assay a three-dimensional cell culture such as a spheroid or organoid. The system comprises a plate body having:
an upper surface;
a lower surface;
at least first and second assay wells each having an open top in fluid communication with the top surface and a transparent base configured to allow imaging of the base of the well from under the plate body; and
at least one fluidic conduit comprising a fluid inlet and a fluid outlet and configured to fluidically connect the fluid inlet, the first well, the second well and the fluid outlet in series.

The top surface of the plate body typically comprises a recessed section having an elongated groove and a cover of resiliently deformable material covering the recess, whereby a first section of the fluidic conduit is defined between the elongated groove and a cover of resiliently deformable material.

In any embodiment, the first recess of the plate body comprises a plurality of fluidic conduits and a plurality of assay wells configured to receive assay fluid from at least one or generally two fluidic conduits.

In any embodiment, the fluidic conduits comprise first fluidic conduits (e.g. at least 2 or 3) and second fluidic conduits (e.g. at least 2 or 3) arranged in an intersecting array having points of intersection and an assay well disposed at least at one of the points of intersection. The provision of intersecting fluidic conduits with assay wells disposed at the points of intersection allows assay fluid to be routed exclusively along one or more rows of wells or one or more columns of wells, using a suitable valving system such as the actuating pins described herein.

In any embodiment, a cell culture model well is located at some, most or every point of intersection of the intersecting array of fluidic conduits.

In any embodiment, the first and second fluidic conduits are generally orthogonal to each other (e.g. they intersect at an angle of 70-90°)

In any embodiment, the first recess of the plate body comprises at least N x M wells arranged in a grid of N columns of wells and N rows of wells, and N first fluidic conduits and M second fluidic conduits, where N and M are each independently at least 3.

In any embodiment, N and M are each independently at least 4.

In any embodiment, N is 4 and M is at least 8, 10 or 12.

In any embodiment, the plate comprises at least 16, 20, 24, 28, 32, 36, 40, 44, or 48 wells.

In any embodiment, the plate comprises at least 16, 20, 24, 28, 32, 36, 40, 44, or 48 fluidic conduits.

In any embodiment, the assay plate system comprises a first fluid control module disposed above the plate body comprising at least one pin and an actuation system coupled to the at least one pin configured to move the pin between a first configuration in which the pin is spaced apart from the plate body and a second configuration in which the pin pushes part of the resiliently deformable cover into the elongated groove to close the fluidic conduit. The fluid control module comprising actuation pins, combined with the resiliently deformable part of the fluidic conduits, provides an efficient valving system for the fluidic conduits (while also providing the option of providing a system for pumping assay fluid throughout he fluidic conduits as described below).

In any embodiment, the first fluid control module comprises at least two pins and the actuation system is configured to independently actuate each pin. This allows the flexibility of closing a selected fluidic conduit.

In any embodiment, the first fluid control module comprises a first set of pins and a second set of pins and the actuation system is configured to independently actuate each set of pins. This allows fluid to be selectively routed in an X-direction or Y-direction across the plate.

In any embodiment, a first pin (or first set of pins) is disposed on a first pin plate and a second pin (or second set of pins) is mounted on a second pin plate, in which the first pin plate comprises an aperture (or apertures) configured to receive the second pin (or set of pins) when the second plate is moved by the actuation system from the first configuration to the second configuration. This provides a suitable arrangement for fitting different sets of actuation pins in a small space over the recessed section of the plate.

In any embodiment, the first pin plate comprises a plurality of first pins and the second pin plate comprises a plurality of second pins, in which the first pin plate comprises a plurality of apertures configured to receive the corresponding second pins.

In any embodiment, the fluid control module comprises:
a frame;
a pin plate mounted to the frame for guided vertical movement relative to the frame; a resiliently deformable element (such as a spring) configured to bias the plate into a raised position;
an axle mounted to the frame for rotation thereon;
a motor operably coupled to the axle to rotate the axle.
a cam mounted to the axle for rotation therewith; and
a cam follower coupled to the pin plate and configured for vertical movement in response to rotation of the cam to lower the plate.

In any embodiment, the frame has upstanding posts and the pin plate has apertures for mounting the plate to the frame for guided vertical movement relative to the plate.

In any embodiment, the or each fluidic conduit comprises at least one valve section defined by a localised depression disposed in the elongated groove configured to receive a pin of the first fluid control module to close the fluidic conduit. The purpose of the depression is to allow a fluid control valve in the form of a pin close the fluidic conduit. The pin is generally disposed above the first plate and is actuatable to push against the resiliently deformable material into the depression to close the fluidic conduit.

In any embodiment, a fluidic conduit fluidically connects a plurality of assay wells and comprises a valve section disposed between each organoid assay well.

In any embodiment, the depression has a depth and/or width that is greater than the depth and/or width of the elongated groove.

In any embodiment, the first fluidic conduits comprise a first set of valve sections configured to restrict flow of assay fluid to the second fluidic conduits when closed, and the first fluid control module comprises a first set of pins configured upon actuation to close the first set of valve sections. This allows the movement of assay fluid across the wells to be controlled to one direction, for example along rows of wells or along columns of wells in series.

In any embodiment, the second fluidic conduits comprise a second set of valve sections configured to restrict flow of assay fluid to the first fluidic conduits when closed, and the first fluid control module comprises a second set of pins configured upon actuation to close the second set of valve sections. This allows the movement of assay fluid across the wells to be controlled to one direction, for example along rows of wells or along columns of wells in series.

In any embodiment, the first set of pins are disposed on a first pin plate and the second set of pins are disposed on a second pin plate and in which the actuation system is configured to move the first and second pin plates independently.

The assay plate system of the invention also includes an assay fluid recirculation system. This is important for the system to reproduce physiological conditions in the wells. The system may include an assay fluid recirculation conduit that recirculated assay fluid from an outlet of a fluidic conduit to an inlet of the fluidic conduit, and an external pumping system. In one preferred embodiment, the system comprises two fluid control modules each having pins configured to engage fluidic conduits, whereby the actuation of the fluid control modules in sequence effects the pumping of fluid through a recirculation conduit.

In any embodiment, the system comprises a fluid recirculation conduit configured to receive assay fluid from the fluid outlet and re-circulate assay fluid to the fluidic inlet of the or each fluidic conduit.

In any embodiment, the top surface of the plate body comprises a second recessed section having an elongated groove and a cover of resiliently deformable material covering the recess, whereby a second section of the fluidic conduit is defined between the elongated groove and a cover of resiliently deformable material, and in which the second section of the fluidic conduit comprises at least one localised depression. In any embodiment, the second recess comprises a second section of a plurality of fluidic conduits.

In any embodiment, the system comprises a second fluid control module disposed above the second recess of the plate body comprising at least one pin and an actuation system coupled to the at least one pin configured to move the pin between a first configuration in which the pin is spaced apart from the plate body and a second configuration in which the pin pushes part of the resiliently deformable cover into the depression in the second section of the fluidic conduit to pump assay fluid into the fluid recirculation conduit.

The provision of a second recess containing a second section of the fluid conduit(s) and a second fluid control module, allows assay fluid to be pumped along the fluidic conduit(s) using the fluid control modules, thereby obviating the need for separate electrical pumps. This is particularly relevant in the case of recirculation of assay fluid around the wells.

In any embodiment, the fluidic conduit comprises a third section providing fluidic communication with the first section and second section.

In any embodiment the third section of the fluidic conduit comprises a section of conduit (e.g. tubing) exposed proud of the plate and comprising a valve actuatable to allow movement of assay fluid from the first section of the fluidic conduit to the second section of the fluidic conduit.

In any embodiment, the valve is a one-way valve. In any embodiment, the valve is configured for actuation by fluid pressure.

In any embodiment, the fluid recirculation conduit comprises a section of tubing exposed proud of the plate and comprising a one-way valve configured to allow movement of assay fluid from the second section of the fluidic conduit to the first section of the fluidic conduit.

In any embodiment, the cover of resiliently deformable material comprises a silicone material.

In any embodiment, the or each cover is a planar layer dimensioned to fit within the recess and fluidically seal the recess.

In any embodiment, the system comprises a well imaging system (e.g. a camera) configured to image the wells of the plate body from underneath the plate body.

In any embodiment, the well imaging system is configured for movement under the plate body to facilitate focussing on an individual well.

In any embodiment, the well imaging system comprises:
a first fluid control module disposed above the plate body comprising at least one pin and an actuation system coupled to the at least one pin configured to move the pin between a first configuration in which the pin is spaced apart from the plate body and a second configuration in which the pin pushes part of the resiliently deformable cover into the elongated groove to close the fluidic conduit; and
a well imaging system configured to image the wells of the plate body from underneath the plate body.

In any embodiment, the well imaging system comprises:
a second recessed section having an elongated groove and a cover of resiliently deformable material covering the recess, whereby a second section of the fluidic conduit is defined between the elongated groove and a cover of resiliently deformable material, and in which the second section of the fluidic conduit comprises at least one localised depression; and
a second fluid control module disposed above the second recess of the plate body comprising at least one pin and an actuation system coupled to the at least one pin configured to move the pin between a first configuration in which the pin is spaced apart from the plate body and a second configuration in which the pin pushes part of the resiliently deformable cover into the depression in the second section of the fluidic conduit to pump assay fluid into the fluid recirculation conduit.

In any embodiment, the plate body comprises a first plate part having well receiving apertures and a second plate part having assay wells with an upper well portion exposed proud of a top of the second plate part, wherein the first and second plate parts are configured to nest together to form the plate body in which the upper well portion of each organoid assay well is disposed within a corresponding well-receiving aperture of the first plate part for receipt of fluid from the fluidic conduit through the open top of the assay wells.

In any embodiment, the plate body comprises:
a first plate part comprising a plate body and a plurality of well-receiving apertures, and a fluidic conduit configured to provide an assay fluid to the well receiving apertures; and
a second plate having a plurality of assay wells each comprising a lower well portion with a well base disposed in the second plate and an upper well portion having an open top defined by an annular sidewall that projects above a top surface of the second plate,
wherein the first and second plates parts are configured to nest together to form the plate body in which the upper well portion of each organoid assay well is disposed within a corresponding well-receiving aperture of the first plate part for receipt of fluid from the fluidic conduit through the open top of the assay wells.

In any embodiment, the first plate part is re-usable and the second plate part is disposable.

In any embodiment, the first plate part comprises at least one, and typically a plurality, of fluid control valve(s) configured to control the flow of fluid to the assay wells through the fluidic conduit. In any embodiment, the fluid control valve is selected from an electrically actuated fluid control valve (e.g. a Burkert valve) and a mechanically actuated fluid control valve.

In any embodiment, the lower part of the second plate part comprises a light transparent material configured to allow imaging of the assay wells by an imaging device from underneath the wells.

In any embodiment, the base of each organoid assay well is formed a light transparent material. In any embodiment, the base of each cell culture model assay well is formed a gas permeable material.

In any embodiment, the sidewalls of each cell culture model well is formed from a material that provides structural rigidity to the second plate, for example an acrylic material.

In any embodiment, the lower part of the second plate part comprises a plurality of well imaging sections comprising light transparent material each having an upper section that forms the base of the well and a lower section that tapers outwardly towards a base of the second plate part.

In any embodiment, the plurality of well imaging sections comprising light transparent material are mounted on, for integrally formed with, a planar base typically formed of light transparent material.

In any embodiment, the well imaging sections are gas permeable.

In any embodiment, the light transparent material comprises PDMS. In any embodiment, the PDMS comprises a curing agent.

In any embodiment, the second plate part comprises a section comprising a shaped recess dimensioned to receive the well imaging sections.

In any embodiment, the plate body comprises a base plate part (optionally detachably) attached to the lower part of the second plate part having a plurality of through-apertures positioned to allow imaging of the base of the assay wells by an imaging device through the well imaging sections.

In any embodiment, the base plate is re-usable.

In any embodiment, at least one and typically all of the assay wells comprise a sidewall that tapers inwardly from the open top towards the well base.

In any embodiment, the well base of one or more assay wells has a diameter D1 and the open top of the assay wells has a diameter D2, wherein a ratio of diameter D1 to D2 is 3:6 to 5:6.

In any embodiment, one or more of the assay wells has a concave base.

In any embodiment, the or each assay well has a depth of 1 to 7 mm.

In any embodiment, an open top of the or each assay well has diameter of 1 - 3.5 mm.

In any embodiment, the base of the or each assay well has diameter of 1 to 3.5 mm.

In any embodiment, the or each assay well has volume of 5-100 µL.

In any embodiment, a lower part of the first plate part comprises a recess dimensioned to receive the second plate part, or an assembly of the second plate part and base plate part, in a nested configuration.

In any embodiment, the base plate part comprises an annular flange comprising a plurality of through apertures configured to receive fixing screws to fix the base plate part to the first plate part.

In any embodiment, the first plate part comprises a recess formed in an upper surface thereof and a top layer of resiliently deformable material dimensioned to nest in the recess.

In any embodiment, the system comprises a sealed chamber in which the assay plate, valve control module(s) and well imaging system are disposed within the sealed chamber. In any embodiment, the system comprises an atmosphere control module to control and modify a parameter of the sealed chamber, for example temperature, gaseous content or humidity.

In any embodiment, the system comprises a processor operatively coupled to the first valve control modules and configured to actuate the first and second plates of the first valve control module in sequence to route assay fluid across the plate in a selected direction, for example in an x-direction or in a y-direction.

In any embodiment, the system comprises a processor operatively coupled to the first and second valve control modules and configured to actuate the first and second valve control modules in sequence to recirculate assay fluid around the fluid conduits.

In another aspect, the invention provides a method of assaying a cell culture model that employs an assay plate system according to the invention, the method comprising the steps of:
Providing an assay plate comprising a cell culture model in an assay well of the assay plate;
providing assay fluid to the assay well containing the cell culture model through the fluidic conduit of the assay plate; and
assaying the response of the cell culture model to the assay fluid.

In any embodiment, the method comprises providing an assay plate having a cell culture model disposed in a plurality of assay wells, providing assay fluid to the assay wells containing the cell culture model through the fluidic conduit of the first plate, and assaying the response of each of the cell culture models to the assay fluid.

In any embodiment, the method comprises a step of growing the or each cell culture model *in-situ* within the assay well prior to the assay step. The method generally comprises adding a precursor cell or cells to the well.

In any embodiment, the step of growing the cells comprising a step of recirculation of assay fluid to the wells of the plate.

In any embodiment, the method comprises a step of assaying the response of the or each cell culture model to a test compound, in which the test compound is added to the assay fluid.

In any embodiment, the response of the or each cell culture model that is assayed is cell viability.

In any embodiment, the assay fluid comprises a reagent configured to allow cell viability of the cell culture model be determined by imaging the well (e.g. a dye).

In any embodiment, the response of the cell culture model that is assayed is protein expression. This may be determined by sampling the fluid in the well and assaying for protein expression by the organoid.

In any embodiment, a first cell culture model or precursor cell is added into a first assay well and a second cell culture model or precursor cell is added into a second assay well.

In any embodiment, the assay plate assembly comprises a column of wells fluidically connected in series in which at least one of the wells of the column contains a cell culture model having a first tissue type (for example, the first well may contain a liver organoid) and at least one other of the wells of the column contains a cell culture model having a second tissue type (for example, the second well may contain a liver organoid).

In any embodiment, the method comprises providing an assay fluid containing a test compound to a first well containing a cell culture model having a first tissue type, and then providing fluid from the first well containing the test compound to a second well containing a cell culture model having a second tissue type.

In any embodiment, the assay plate assembly comprises at least nine assay wells arranged in a grid comprising N columns and M rows in which N and M are each independently 3 or greater.

In any embodiment, the assay plate assembly comprises a first fluidic conduit configured to supply assay fluid to one or more of the N columns of wells in which the method comprises supplying assay fluid to the wells of one or more of the columns of wells in series.

In any embodiment, the assay plate assembly comprises a second fluidic conduit configured to supply assay fluid to one or more of the M rows of wells in which the method comprises supplying assay fluid to the wells of one or more of the rows of wells in series.

In any embodiment, the cell culture model is selected from a 2-D cell culture model and a 3-D cell culture model. In any embodiment, the 3-D cell culture model is selected from an organoid, and/or a microtissue and/or a spheroid.

Other aspects and preferred embodiments of the invention are defined and described in the other claims set out below.

### Brief Description of the Figures

**FIG. 1** is a top plan view of an assay plate of the invention.
**FIG.2** is an end elevational view of the assay plate of Figure 1.
**FIG. 3** is a detailed view of an assay well and two fluidic conduits of the assay plate of Figure 1.
**FIG.4** is a detailed view of a depression forming part of the second recess of the assay plate of Figure 1.
**FIG. 5** is a sectional view of part of the assay plate of Figure 1 showing adjacent assay wells and fluidic conduit with a depression fluidically connecting the top of the wells.
**FIG. 6** is a sectional view taken along the lines A-A of Figure 1.
**FIG.7A** is a perspective view from above of a second plate part forming part of one embodiment of an assay plate of the invention.
**FIG. 7B** is a perspective view from below of the second plate part of Figure 7A.
**FIG.7C** is a side elevational view of the second plate part of Figure 7A.
**FIG.8A** is a perspective view from above of a base plate part forming part of one embodiment of the assay plate of the invention.
**FIG. 8B** is a perspective view from below of the base plate part of Figure 8A.
**FIG.8C** is a side elevational view of the base plate part of Figure 8A.
**FIG. 9** is a sectional view of the assay plate of the invention showing the assay wells and first section of a fluidic conduit disposed in the first recess, second recess with second section of the fluidic conduit, third section of the fluidic conduit and fluid recirculation conduit.
**FIG. 10** is a perspective view showing the assay plate system of the invention including plate body with first and second recesses and a first fluid control module positioned over the first recess and second fluid control module positioned over the second recess with pins engaged into the depressions of the second recess.
**FIG. 11A** is a side elevational view of an assay plate system of Figure 10 showing the actuation system for the first fluid control module positioned and second fluid control module.
**FIG. 11B** is a sectional view taken along the line E-E of Figure 11A.
**FIG. 11C** is a sectional view taken along the line F-F of Figure 11A.
**FIG. 12A** is an end elevational view of an assay plate system of Figure 10 showing the actuation system for the first fluid control module positioned and second fluid control module.
**FIG. 12B** is a sectional view taken along the line E-E of Figure 12A.
**FIG. 12C** is a sectional view taken along the line F-F of Figure 12A.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

The cell culture model may be a 2-D or 3-D cell culture model. As used herein, the term "3-D cell culture model" refers to a miniaturized and simplified version of an organ produced in vitro in three dimensions that shows realistic micro-anatomy. Examples include organoids, microtissues and spheroids. Organoids/microtissues are derived from one or a few cells from a tissue, embryonic stem cells or induced pluripotent stem cells, which can self-organize in three-dimensional culture owing to their self-renewal and differentiation capacities. Such 3-D cell culture models typically has a maximum dimension of 50-1500 µm. Examples of 3-D cell culture model include liver, hepatic, pancreatic, epithelial, kidney, cardiac, retinal, blastoid, glioblastoma, thyroid and testicular organoids.

As used herein, the term "3-D cell culture model precursor" or "precursor" refers to a cell or composition of cells capable of being cultured to form a 3-D cell culture model. The precursor may be a cell from a specific tissue, a cell line, a patient cell or tissue sample, an embryonic stem cell, or an induced pluripotent stem cell. The assay plate system of the invention may be used to assay cell culture models such as organoids and to grow 2-D or 3-D cell culture model from precursor cells. Growth generally comprises placing a suitable precursor cell type(s) into a well, adding assay fluid (which may be cell culture fluid containing agents to promote the growth of the desired 2-D or 3-D cell culture model), and recirculation of the assay fluid to the well or wells.

As used herein, the term "assay plate system" refers to an assay plate body having assay wells suitable for assaying a 2-D or 3-D cell culture model such as an organoid and one or more fluidic conduits fluidically connecting at least two wells, and optionally a fluid control module comprising at least one pin configured to engage upon actuation a fluidic conduit, and optionally a well imaging system to image the wells of the plate from underneath the plate. The plate is generally planar and the wells are formed in the top of the plate, generally in a recess formed in the top of the plate. The plate may be a unitary structure or it may be formed by two plate parts that nest together to form the plate. When the plate is formed in two parts, the wells are generally provided in the second plate part have an upper well part that projects proud of a surface of the second plate part. The first plate comprises apertures for receiving the wells of the second plate in a fluidically tight manner. The wells of the assay plate body generally have a base formed of a light transparent material that allows the wells to be imaged with an imaging system disposed under the plate.

As used herein, the term "disposable" as applied to the second plate part means that the second plate part is not re-usable and is detached from the first plate part and disposed of after use, allowing the first plate part to be re-used with a replacement second plate.

As used herein, the term "light transparent material" refers to a material that is transparent. Sections of the lower part of the second plate, including the base of the wells, is generally formed from a light transparent material allow the organoids in the wells to be imaged with an imaging device from below the second plate. The light transparent material is generally a polymeric material suitable for melting and casting. The light transparent material may be PDMS, cyclicolefin copolymer (COC), perfluoropolyethers (PFPEs), polyurethane, Flexdym, polylactic acid (PLA).

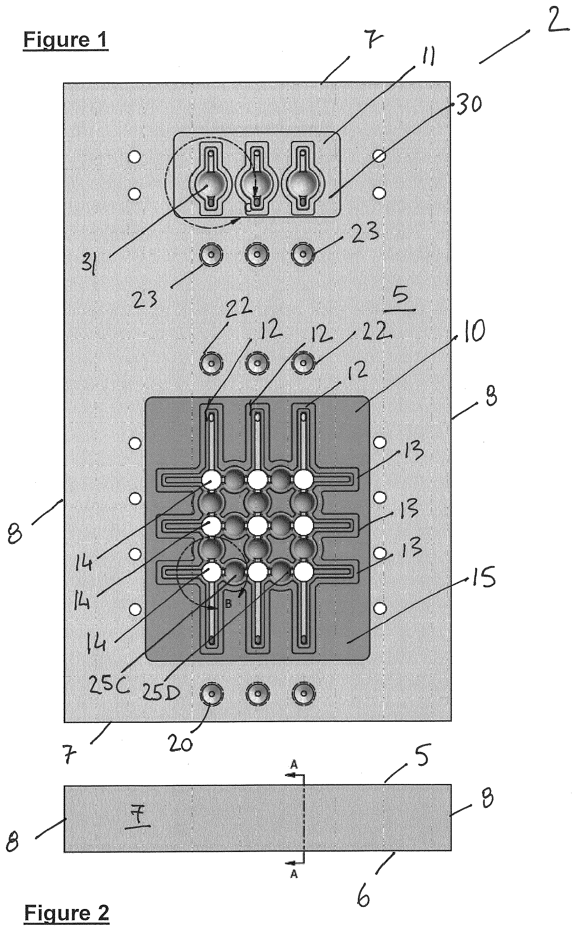

As used herein, the term "intersecting array" as applied to the fluidic conduits refers to an arrangement of first fluidic conduits (for example 2 or more) that extend in a first orientation and second fluidic conduits that extend in a second orientation and intersect the first fluidic conduits fluidic conduits, with an assay well disposed at some or all of the points of intersection. This is illustrated in Figure 1. The term "N x M grid assembly" as applied to the wells of the plate body refers to a plurality of wells arranged in a grid format comprising M columns and N rows. Generally M and N are each, independently, a whole number greater than 2, for example 3, 4, 5, 6, 7, 8, 9, 10, 11 or 12.

As used herein, the term "well imaging system" refers to an imaging device configured to image the wells of the plate assembly, generally from a position beneath the plate assembly. Examples of imaging devices include microscopes that can undertake white light and fluorescent imaging.

As used herein, the term "fluid control module" refers to a part of the system of the invention that is actuatable to open and close a fluidic conduit of the plate body. The module generally comprises at least one pin and an actuation system for the pin to move the pin from a first position spaced apart from the plate assembly to a second position in which the pin causes the fluidic conduit to close. The fluid control valve module generally has a plurality of pins, for example at least 8, 18, 32, or 50. Generally the pins comprise a first set of pins that actuate together, and a second set of pins that actuate together, in which the actuation system is configured to actuate the first set of pins independently of the second set of pins. In one embodiment, the first set of pins are mounted to a first plate, the seconds set of pins are mounted to a second plate disposed above the first plate, and the first plate comprises a plurality of apertures corresponding to the second set of pins and configured to allow the second set of pins project through the first plate when the second plate is moved towards the first plate. The actuation system is operatively coupled to the first and second plates and configured to move each plate independently towards the top surface of the first plate.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

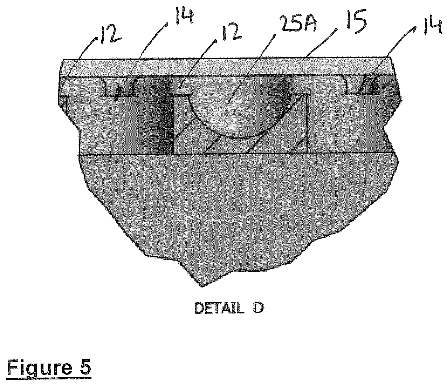

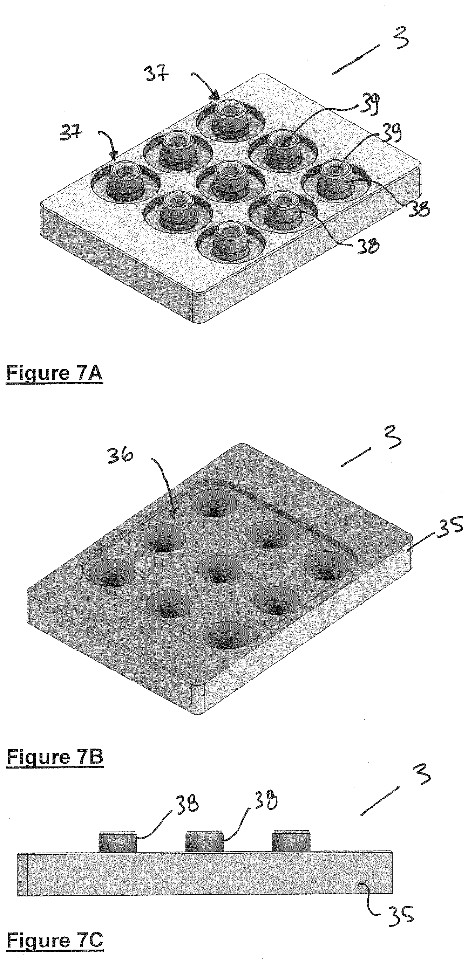

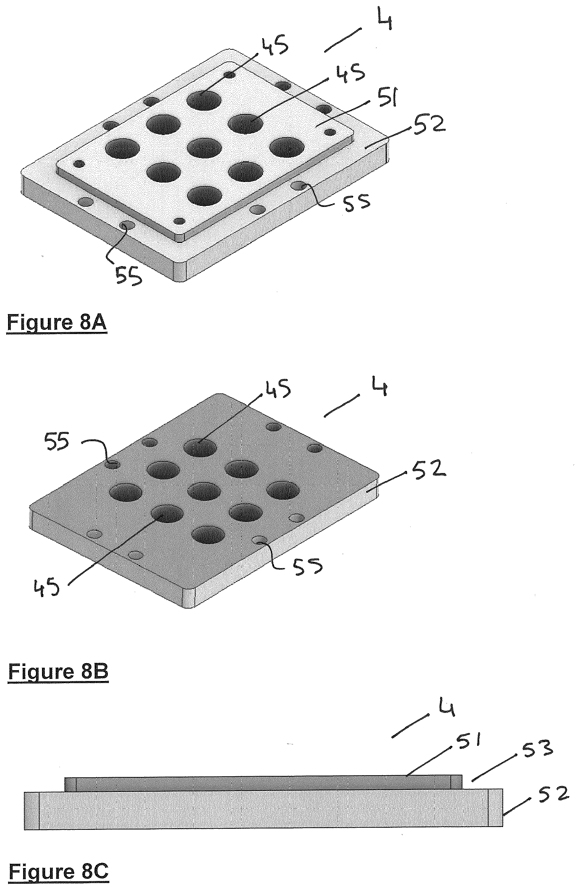

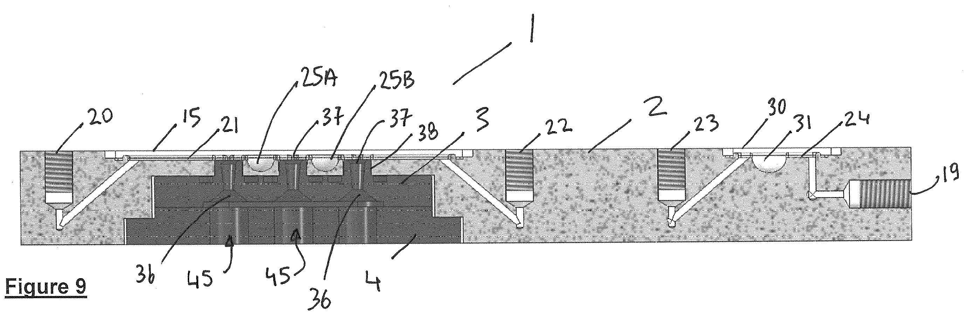

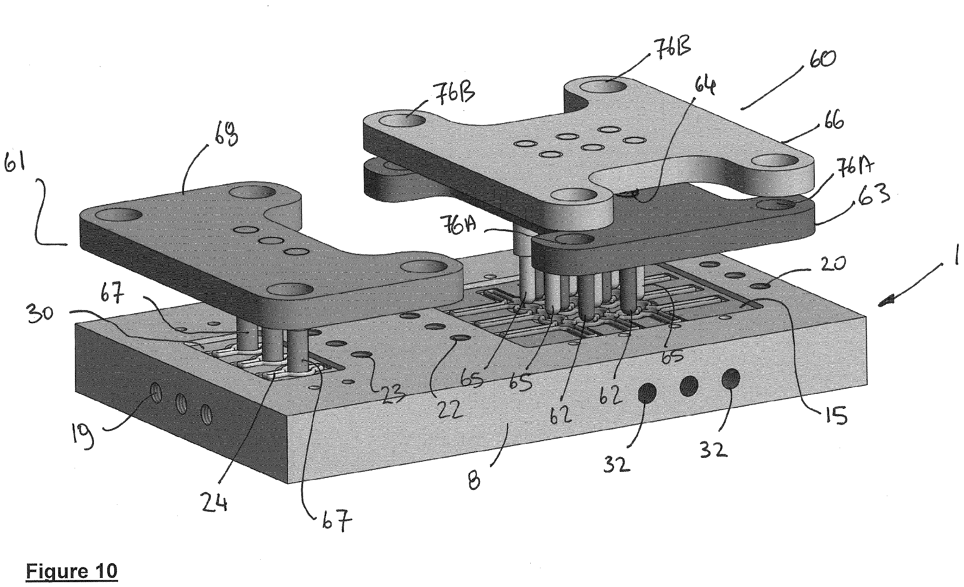

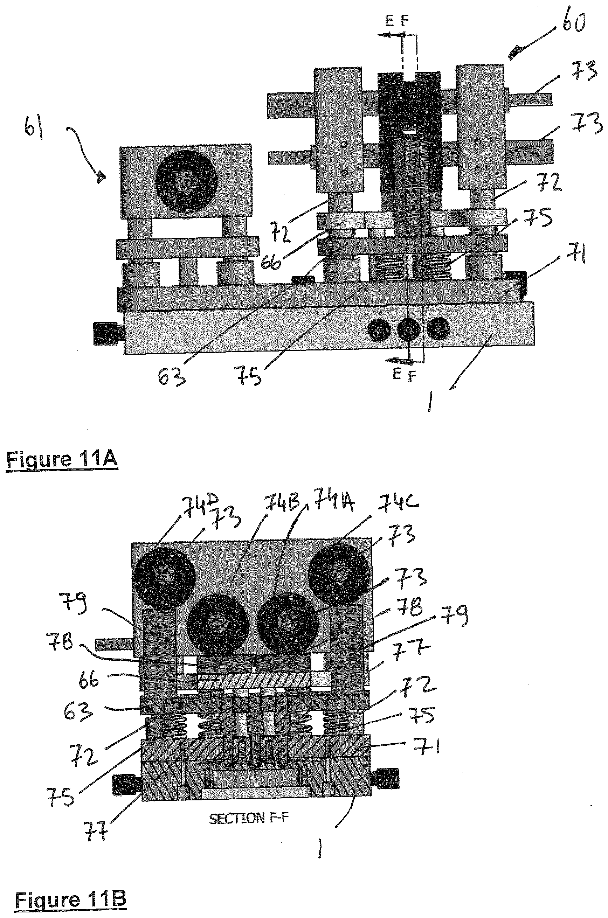

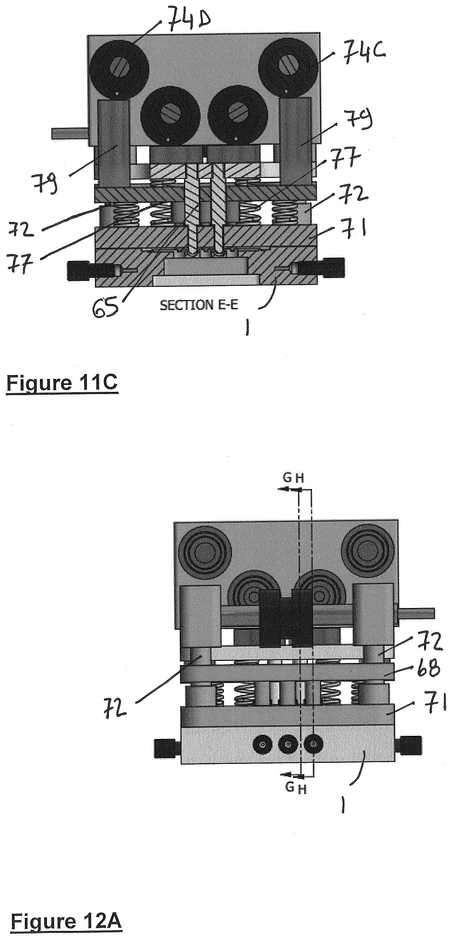

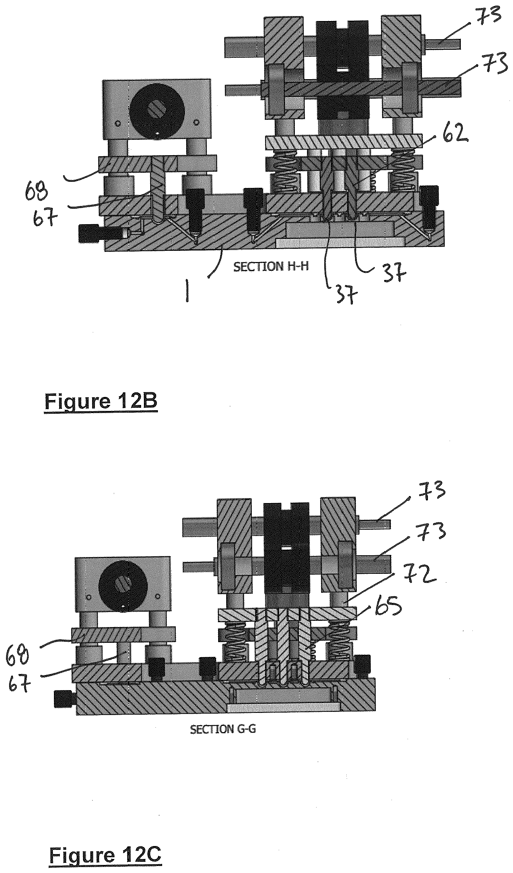

Referring to the drawings, an assay plate system of the invention is described in detail. In the embodiment describes, the system comprises an assay plate 1 formed from three parts that nest together to form the plate body, namely a first (upper) plate part 2 illustrated in Figures 1 to 6, a second plate part 3 illustrated in Figures 7A to 7C, and a base plate part 4 illustrated in Figures 8A to 8C. The provision of wells in the second plate part allows the second plate part to be disposable, allowing re-use of the first plate part which does not come into direct contact with the 3-D cell culture model that is being assayed. Figure 9 illustrated the assembled plate body. Figure 10 illustrates the first fluid control module disposed over the assay wells in the plate, and Figure 11 illustrates an assay fluid recirculation system comprising the first fluid control module disposed over the assay wells disposed in the first recess and a second fluid control module disposed over fluid pumping wells disposed in a second recess in the plate. Figure 12 illustrates one full fluidic circuit comprising a fluidic conduit and fluid recirculation conduit, and a fluid inlet and fluid outlet. It will be appreciated that the plate body does not need to be formed in separate parts but may be formed from a single plate body.

Referring to Figures 1 to 6, the first plate part 2 is described and comprises a planar plate having a top surface 5, lower surface 6, end walls 7 and sidewalls 8. The top surface 5 has a first recessed section 10 and a second recessed section 11. The first recessed section 10 comprises three first fluidic conduits 12 (Y-conduits) and three second fluidic conduits 13 (X-conduits) that intersect at through apertures 14. The apertures receive wells provided on a second plate part as described below during assembly of the plate such that the top of the wells is provided on the same plane as the fluidic conduits. When assembled the first recessed section has nine wells arranged in a 3 x 3 grid array. The fluidic conduits are defined along part of their length by elongated grooves formed in the top surface of the first recessed section 10 and a silicone layer 15 that covers the top of the recess, fluidically sealing the recess and forming an upper surface of the fluidic conduits. In this manner, the fluidic conduits can be deformed and pinched closed using pins of a fluid control module as described in more detail below.

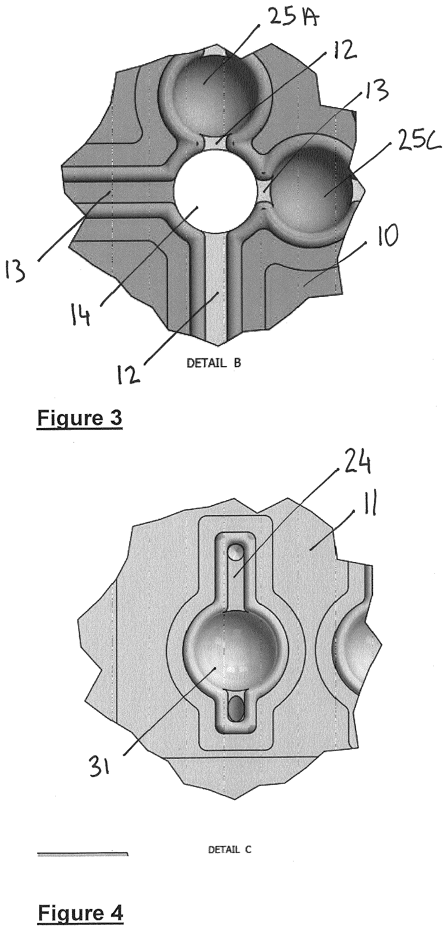

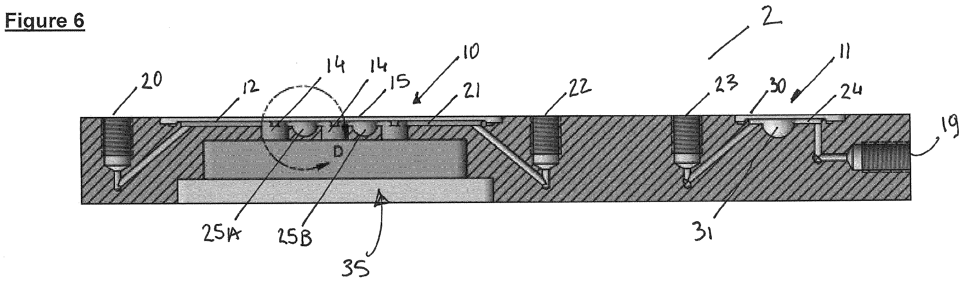

Each of the first fluidic conduits 12 (Y-conduits) has a fluid inlet 20 on the top of the plate, a first section 21 disposed in the first recessed section 10, an outlet 22 on the top of the plate, an inlet 23 on the top of the plate, a second section 24 disposed in the second recessed section 11, and a fluid outlet 19 disposed in the end wall 7 (See Figure 6). The first 21 section of the fluidic conduit provides fluidic communication to a column of three wells (disposed in the apertures 14 when the plate is assembled, as illustrated in Figure 9) in series and includes a first depression 25A disposed between the first and second apertures and a second depression 25B disposed between second and third apertures. Referring specifically to Figure 3, a section of the first recessed section 10 of the plate is illustrated showing a first fluidic conduit 12 intersecting with a second fluidic conduit 13 at a well-receiving aperture 14 and a depression 25A disposed between the first and second wells in the column of wells. The depression 25A has a width and depth greater than the width and depth of the fluidic conduits allowing a pin forming part of a fluid control module to press the overlying silicone layer into the depression to close the first fluidic conduit (as described in more detail below).

The second section 24 of the first fluidic conduits 12 is provided in the second recessed section 11 and defined by an elongated groove and a second silicone layer 30 that covers the top of the recess 11, fluidically sealing the recess and forming an upper surface of the second section of the fluidic conduits. The second section 24 also includes a fluid pumping well 31 that in use receives a pin forming part of a second fluid control module to press the overlying silicone layer into the well to pump fluid in a circuit around the plate.

Each of the three second fluidic conduits 13 (X-conduits) comprises a fluid inlet 32 disposed in a sidewall 8 of the plate, a fluid outlet (not shown) disposed in an opposite sidewall of the plate, and a central section disposed in the recessed section of the plate 10 (defined as described for the first fluidic conduit by an elongated groove and cover of silicone) that provides fluidic communication to a column of three wells (disposed in the apertures 14 when the plate is assembled, as illustrated in Figure 9) in series. Each of the second fluidic conduits 13 includes a first depression 25C disposed between the first and second apertures in the row and a second depression 25D disposed between second and third apertures in the row.

Referring to Figure 6, the lower surface of the plate comprises a stepped recess 35 dimensioned to receive the second plate part 3 and base plate part 4. The assembled plate with the second plate part 3 and base plate part 4 nested in the recess 35 is illustrated in Figure 9.

Referring to Figures 7A to 7C, the second plate part 3 has a rectangular planar plate body comprising a first plate section 35 formed of acrylic and a lower well imaging section 36 formed of light transparent and gas diffusing PDMS. Nine wells 37 are provided each having an upper well section 38 projecting proud of the top surface of the plate. Each well has a depth of about 6.75 mm with an open top 39 having a diameter of about 2.89 mm and a concave base 40 having a diameter across the top of the concave part of about 2.04 mm. The sidewalls of each well 37 are defined by the acrylic first plate section 35. The PDMS well imaging section 36 has a planar base and a plurality of frustoconical sections 43 that extend upwardly with inwardly tapering sidewalls from the planar base to form the concave base 40 of the wells. The base plate has apertures 45 in the base that are aligned with the frustoconical PDMS sections 36 to allow the wells to be viewed with an imaging device (not shown) disposed underneath the plate assembly 1. The PDMS also allows for gas diffusion into the wells.

Referring to Figures 8A to 8C, the base plate 4 is illustrated and comprises a planar rectangular plate 50 having a top part 51, bottom part 52, stepped sidewall 53, and nine apertures 45 extending through the plate from the top surface to the bottom surface. The top part 51 is dimensioned to align with the base of the second plate part 3 when the second plate part and base plate part are coupled together. Referring to Figure 3, the bottom part comprises holes 55 to receive fixing screws for fixing the base plate part 4 in the recess 35 of the first plate part 2.

Referring to Figure 10, an assay plate system according to one embodiment of the invention is illustrated in which parts described with reference to the previous embodiments are assigned the same reference numerals. The system comprises an assay plate 1 as described with reference to Figures 1 to 9, a first fluid control module 60 positioned above the 3 x 3 grid array of wells 37 disposed in the first recessed section 10 of the plate, and a second fluid control module 61 positioned above the second recessed section 11 of the plate. The actuation system for the fluid control modules is removed for clarity. The first fluid control module 60 comprises a first set of pins 62 attached to a first plate 63 having a plurality of apertures 64, and a second set of pins 65 that are longer than the first set of pins attached to a second plate 66 disposed above the first plate 63, with the second set of pins projecting through the apertures of the first plate 63. The second fluid control module 61 comprises three pins 67 attached to a third plate 68.

The first set pins 62 are positioned over the depressions 25A, 25B in the first fluidic conduits 12 and the second set of pins 65 are positioned over the depressions 25C, 25D in the second fluidic conduits 13. A plate actuation means (illustrated in Figures 11 and 12) is operatively coupled to each plate and configured to move each plate independently towards the top of the assay plate 1. When the second plate 66 is moved towards the assay plate 1 and the first plate 63 is not moved, the second set of pins 65 are depressed pushing the silicone cover into the depression 25C, 25D in the second fluidic conduits 13 closing those conduits and preventing assay fluid moving across the rows of wells of the plate in a X-direction, with the result that flow of assay fluid is channelled across the columns of wells via the first fluidic conduits 12 in a Y-direction. Similarly, when the first plate 63 is moved towards the assay plate 1 and the second plate 66 is not moved, the first set of pins 62 are depressed pushing the silicone cover into the depressions 25A, 25B of closing the first fluidic conduits 12 and preventing assay fluid moving across the wells of the plate in a Y-direction, with the result that flow of assay fluid is channelled across the column of wells via the X-direction conduits.

Figures 11A to 11C and 12A to 12C illustrate the actuation system for the first fluid control module 60 and second fluid control module 61 in more detail. The actuation system for the first fluid control module 60 comprises a frame 70 having a base plate 71 with apertures, upright supporting arms 72 and four axles 73 mounted between the arms. Each axle supports an eccentrically mounted camwheel 74. The first plate 63 is mounted to the arms 72 which are received by apertures 76A to allow vertical sliding movement on the plate of the arms and is spaced apart from the base plate 72 by first springs 75. The second plate 66 is similarly mounted to the arms received in apertures 76B for vertical sliding movement and spaced apart from the base plate 72 by second springs 77. The springs bias the plates away from the assay plate 1. The camwheels 74A and 74B are operably connected to the second plate 66 by cam followers 78 so that when the cam wheels rotate the second plate is urged downwardly pushing the second set of pins 65 into engagement with the silicone cover and fluidic conduits as illustrated in Figure 11C and 12C. The camwheels 74C and 74D are operably connected to the first plate 63 by cam followers 79 so that when the cam wheels rotate the first plate is urged downwardly pushing the first set of pins 62 into engagement with the silicone cover and fluidic conduits as illustrated in Figure 11B and 12B. Although the springs and cam followers are not illustrated second fluid control module 61 is of broadly the same construction as the first fluid control module.

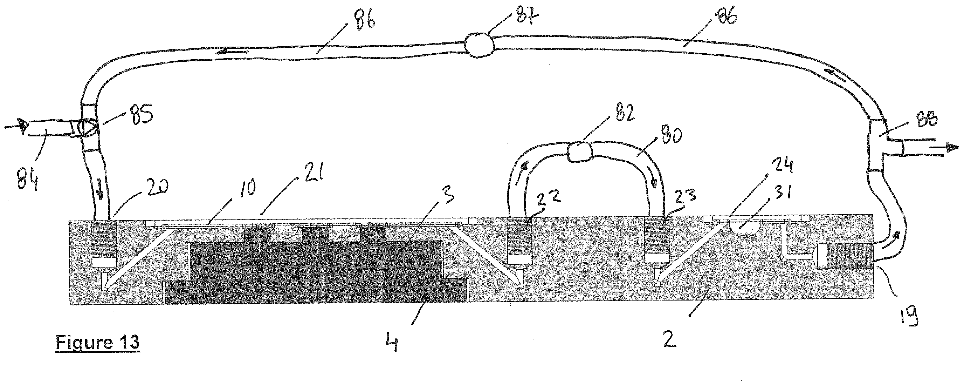

Referring to Figure 13, the fluid recirculation system of the assay plate 1 is described in more detail. One first fluidic conduit is illustrated and comprises a fluid inlet 20 on the top of the plate, a first section 21 disposed in the first recessed section 10, an outlet 22 on the top of the plate, an inlet 23 on the top of the plate, a second section 24 disposed in the second recessed section 11, and a fluid outlet 19 disposed in the end wall 7 (See Figure 6). An external conduit 80 having a pressure activated one-way valve 82 is shown fluidically connecting the outlet 22 and inlet 23. An assay fluid supply conduit 84 provides fluid from a fluid reservoir (not shown) to an inlet divert valve 85, which is operable to fluidically connect the fluid supply conduit 84 with the fluid inlet 20 (to supply assay fluid to the plate) or to fluidically connect the fluid inlet 20 with a fluid recirculation conduit 86 to receive assay fluid that is being recirculated from the fluid outlet 19. The fluid recirculation conduit 86 also includes a pressure activated one-way valve 87. A second divert valve 88 is provided and operable to direct the assay fluid to waste or to the recirculation conduit.

In use, and referring to Figures 13 and 10, the sequential operation of the fluid control modules works to recirculate assay fluid across the wells via the first fluidic conduits. In a first step, the pins of the second fluid control module are depressed into engagement with the pumping wells of the second recessed section of the plate, and the pins of the first fluid control module are spaced from the plate, as shown in Figure 10. Due to the presence of the one-way valve 82 the assay fluid is forced into the fluid recirculation conduit 86 where it passes through the second pressure activated one-way valve 87. In a second step, the pins of the second fluid control module are retracted while the pins of the first fluid control module are depressed. This pushes assay fluid from the first section of the first fluidic conduits into the external conduit through the one-way valve 82 and simultaneously the retraction of the pins of the second fluid control module pulls assay fluid into the second section of the first fluidic conduits. Repeating this sequential operation effects the movement of assay fluid through the first fluidic conduits and wells and into the recirculation conduit, causing the recirculation of assay fluid across the wells of the plate.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. An assay plate system to assay a 2-D or 3-D cell culture model comprising an assay plate (1) having:
an upper surface (5);
a lower surface;
at least first and second assay wells (37) each having an open top (39) in fluid communication with the upper surface and a transparent base (40) configured to allow imaging of the base of the well from under the plate body; and
at least one fluidic conduit (12, 13) comprising a fluid inlet and a fluid outlet and configured to fluidically connect the fluid inlet, the first well, the second well and the fluid outlet in series,
wherein the upper surface of the assay plate comprises a recessed section (10, 11) having an elongated groove and a cover of resiliently deformable material (15, 30) covering the recess, whereby a first section of the fluidic conduit is defined between the elongated groove and a cover of resiliently deformable material.

2. An assay plate system according to Claim 1, in which the first recess (10) of the assay plate comprises a plurality of first fluidic conduits (12) and a plurality of second fluidic conduits (13) arranged in an intersecting array, in which an assay well (37) is located at each point of intersection of the intersecting array of fluidic conduits.

3. An assay plate according to Claim 2, in which the first recess (10) of the assay plate comprises at least N x M wells (37) arranged in a grid of N columns of wells and N rows of wells, and N first fluidic conduits (12) and M second fluidic conduits (13), where N and M are each independently at least 3.

4. An assay plate system according to any preceding Claim, comprising a first fluid control module (60) disposed above the assay plate comprising at least two pins (62,65) and an actuation system coupled to the at least two pins configured to move the pins between a first configuration in which the pins are spaced apart from the assay plate and a second configuration in which each pin pushes part of the resiliently deformable cover (15) into an elongated groove to close a fluidic conduit (12) and the actuation system is configured to independently actuate each pin plate.

5. An assay plate system according to Claim 4, in which a first pin or plurality of first pins (62) is disposed on a first pin plate (63) and a second pin or plurality of second pins (65) is mounted on a second pin plate (66), in which the first pin plate comprises one or more apertures (64) configured to receive the second pin or pins when the or each second pin is moved by the actuation system from the first configuration to the second configuration.

6. An assay plate system according to any of Claims 4 or 5, in which one or more of the fluidic conduits (12, 13) comprises at least one valve section defined by a localised depression (25A, 25B, 25C, 25D) disposed in the elongated groove, in which the pin (62, 65) of the fluid control valve module is configured to push part of the resiliently deformable cover (15, 30) into the localised depression to fluidically close the fluidic conduit.

7. An assay plate system according to Claim 5 or 6, in which the first fluidic conduits (12) comprise a first set of valve sections configured to restrict flow of assay fluid to the second fluidic conduits (13) when closed, and the first fluid control module (60) comprises a first set of pins (62) configured upon actuation to close the first set of valve sections.

8. An assay plate system according to any of Claims 2 to 7, in which the second fluidic conduits (13) comprise a second set of valve sections configured to restrict flow of assay fluid to the first fluidic conduits (12) when closed, and the first fluid control module (60) comprises a second set of pins (65) configured upon actuation to close the second set of valve sections.

9. An assay plate system according to Claim 8, in which the first set of pins (62) are disposed on a first pin plate (63) and the second set of pins (65) are disposed on a second pin plate (66) and in which the actuation system is configured to move the first and second pin plates independently.

10. An assay plate according to any preceding Claim, including a fluid recirculation conduit (86) configured to receive assay fluid from the fluid outlet (19) and re-circulate assay fluid to the fluidic inlet (20) of the or each fluidic conduit (12).

11. An assay plate system according to Claim 10, in which the upper surface (5) of the assay plate (1) comprises a second recessed section (11) having an elongated groove and a cover of resiliently deformable material (30) covering the recess, whereby a second section of the fluidic conduit (12) is defined between the elongated groove and a cover of resiliently deformable material, and in which the second section of the fluidic conduit comprises at least one localised depression (31).

12. An assay plate system according to Claim 11, including a second fluid control module (61) disposed above the second recessed section (11) of the assay plate comprising at least one pin (67) and an actuation system coupled to the at least one pin configured to move the pin between a first configuration in which the pin is spaced apart from the plate body and a second configuration in which the pin pushes part of the resiliently deformable cover into the depression (31) in the second section of the fluidic conduit (12) to pump assay fluid into the fluid recirculation conduit (86).

13. An assay plate system according to any of Claims 11 or 12,
in which the fluidic conduit comprises a third section providing fluidic communication with the first section and second section and comprising a section of tubing (80) exposed proud of the assay plate (1) and comprising a one-way valve (82) configured to allow movement of assay fluid from the first section of the fluidic conduit to the second section of the fluidic conduit and,
in which the fluid recirculation conduit (86) comprises a section of tubing exposed proud of the plate and comprising a one-way valve (87) configured to allow movement of assay fluid from the second section of the fluidic conduit to the first section of the fluidic conduit.

14. An assay plate system according to Claim 1, and comprising:
a first fluid control module (60) disposed above the assay plate (1) comprising at least one pin (62) and an actuation system coupled to the at least one pin configured to move the pin between a first configuration in which the pin is spaced apart from the assay plate and a second configuration in which the pin pushes part of the resiliently deformable cover (15) into the elongated groove to close the fluidic conduit (12); and
a well imaging system configured to image the wells (37) of the assay plate (1) from underneath the assay plate.

15. An assay plate system according to any preceding Claim, in which the assay plate comprises a first plate body part (2) having well receiving apertures (14) and a second plate body part (3) having assay wells (37) with an upper well portion (38) exposed proud of a top of the second plate body part, wherein the first and second plate body parts are configured to nest together to form the assay plate in which the upper well portion of each assay well is disposed within a corresponding well-receiving aperture of the first plate for receipt of fluid from the fluidic conduit through the open top of the assay wells.
